# EUROPEAN PATENT SPECIFICATION

(11) **EP 2 558 870 B1**
(45) Date of publication and mention of the grant of the patent: **07.06.2017**
(21) Application number: 11768557.8
(22) Date of filing: 11.04.2011
(51) Int. Cl.: G01N 35/00, G01N 33/48, G01N 21/62, G01N 21/76, G01N 21/64, G01N 33/50

(54) **SYSTEM AND METHOD FOR RAPID DETERMINATION OF A MEDICAL CONDITION**
VORRICHTUNG UND VERFAHREN ZUR SCHNELLEN BESTIMMUNG EINES MEDIZINISCHEN LEIDENS
DISPOSITIF ET PROCÉDÉ POUR DÉTERMINER RAPIDEMENT UN ÉTAT PATHOLOGIQUE

(30) Priority: 15.04.2010 US 324315 P
(43) Date of publication of application: 20.02.2013
(73) Proprietor: Leukodx Ltd, Jerusalem 91451 (IL)
(72) Inventor: KASDAN, Harvey, Lee, Jerusalem 91451 (IL); RAJUAN, Menashe, Jerusalem 94352 (IL); BRODER, Yehoshua, Jerusalem 91451 (IL); MEISSONNIER, Julien, Jerusalem 91451 (IL); DAVIS, Bruce, Bangor, Maine 04401 (US); ZUTA, Yoav, Jerusalem 91451 (IL)
(74) Representative: Becker Kurig Straus
(86) International application number: PCT/IL2011/000296
(87) International publication number: WO 2011/128893

(56) References cited:
- WO-A2-2006/055816
- US-A- 4 730 899
- US-A1- 2003 194 752
- US-A1- 2004 155 309
- US-A1- 2005 105 077
- US-A1- 2005 275 839
- US-A1- 2008 212 102
- US-B2- 6 897 954
- SVEN BJÖRNSSON ET AL: "Total nucleated cell differential for blood and bone marrow using a single tube in a five-color flow cytometer", CYTOMETRY. PART B, CLINICAL CYTOMETRY, vol. 74B, no. 2, January 2008 (2008-01), pages 91-103, XP055251112, US ISSN: 1552-4949, DOI: 10.1002/cyto.b.20382
- HAMMATSU PHOTONICS K.K.: 'Electron Tube Division.' PHOTON COUNTING USING PHOTOMULTIPLIER TUBES July 2005, XP008170237 Retrieved from the Internet: <URL:http://sales.hamamatsu.com/assets/appl ications/ETD/PhotonCounting_TPH09001E04.pdf > [retrieved on 2011-09-13]

## Description

### FIELD OF THE INVENTION

The present invention relates to the field of human diagnostics, in general, and to fluorescent-based analysis of cell-associated biological markers, in particular.

### BACKGROUND OF THE INVENTION

There is a great requirement for fast and accurate information in the field of medical diagnostics. It is generally preferred that therapeutic agents and overall patient treatment are not applied until actionable patient diagnostic information is available. Numerous methodologies have been developed for identifying causative agents for a vast number of ailments. PCR, ELISA, flow-cytometry, lateral flow, and other methods are routinely used for identifying pathogenic species associated with human illness.

Sepsis is an extreme condition resulting from body responses to a systemic inflammatory response syndrome (SIRS) caused by a pathogenic agent. Sepsis may often lead to massive organ failure and death. Sepsis is generally identified by general patient features such as body temperature, heart rate, respiratory rate, and white blood count. Of late, several groups have identified markers or combinations of biological markers that may serve to identify sepsis occurring in a patient. Knowing that a patient is in a septic state or may be heading towards a septic response to a bacterial, viral, or fungal infection is critical for proper patient management and drug selection.

US Patent 4,745,285, to Recktenwald and Chen, teaches a method for determining one or more characteristics of particles using multiple fluorescence analysis, which involves directing an incident light beam at the particles under analysis. The particles include at least three fluorescent markers each having different emission spectra. The incident light beam causes the excitation of the markers by light at a single wavelength whereby different wavelengths of fluorescence are emitted from the particles. Different fluorescence emissions associated with the particles under analysis are simultaneously detected. This method further includes associating the detected fluorescence with one or more characteristics of the particles. An apparatus is also part of the present invention for carrying out the aforementioned method.

U.S. Patent 5,627,040, to Bierre and Mikaels, teaches a method for autoclustering N-dimensional datastreams. The invention has particular utility in analyzing multi-parameter data from a flow-cytometer, and more particularly has utility in analyzing data from whole blood cells tagged with fluorescently labelled CD3, CD4, and CD8 monoclonal antibodies, to which a known number of fluorescent microbeads have been added.

U.S. Patent 6,636,623, to Nelson et al., describes a system and method for rapidly detecting cells associated with malignancy and disease using molecular marker compartmentalization includes an optical tomography (OT) or a flow optical tomography (FOT) instrument capable of producing various optical projection images (or shadowgrams) containing accurate density information from a cell or cells labelled with tagged molecular probes or stains, a computer and software to analyze and reconstruct the projection images into a multi-dimensional data set, and automated feature collection and object classifiers. The system and method are particularly useful in the early detection of cancers, such as lung cancer, using cells from sputum or cheek scrapings, and cervical/ovarian cancer using a cervical scraping. The system may be used to detect rare cells in specimens including blood.

U.S. Patent 7,024,316 to Ellison, et al. describes a flow cytometry apparatus and methods to process information incident to particles or cells entrained in a sheath fluid stream allowing assessment, differentiation, assignment, and separation of such particles or cells even at high rates of speed. A first signal processor individually or in combination with at least one additional signal processor for applying compensation transformation on data from a signal. Compensation transformation may involve complex operations on data from at least one signal to compensate for one or numerous operating parameters. Compensated parameters may be returned to the first signal processor for provide information upon which to define and differentiate particles from one another.

U.S. Patent Application 20050255600, to Padmanabhan et al., describes a sample analyzer cartridge for use at a point of care of a patient such as in a doctor's office, in the home, or elsewhere in the field. By providing a removable and/or disposable cartridge with all of the needed reagents and/or fluids, the sample analyzer may be reliably used outside of the laboratory environment, with little or no specialized training.

International Patent Application WO 2006/0055816, to Davis et al., describes a method of quantifying CD64 and CD163 expression in leukocytes and, specifically to a kit for use with a flow cytometer, including a suspension of quantitative fluorescent microbead standards, fluorescent labelled antibodies directed to CD64 and CD 163, and analytical software. The software is used to take information on the microbead suspension and fluorescent labelled antibodies from a flow cytometer and analyze data, smooth curves, calculate new parameters, provide quality control measures and notify of expiration of the assay system.

International Patent Application WO 2008/124,589, to Tai et al., describes particular embodiments relating to a microfluidic device that may be utilized for cell sensing, counting, and/or sorting. Particular aspects relate to a microfabricated device that is capable of differentiating single cell types from dense cell populations. One particular embodiment relates a device and methods of using the same for sensing, counting, and/or sorting leukocytes from whole, undiluted blood samples.

International Patent Application WO2009/003493, to Jacobsen et al., teaches methods for assaying an entity present in a sample. The method comprises a number of individual steps, such as the steps of obtaining a sample, preparing the sample for assaying, and assaying the sample. The method may comprise further optional processing steps associated with either sample processing or processing of data obtained as a result of having assayed the sample. The sample may initially be assayed e.g. for presence of one or more entities. The step of assaying the sample may further comprise the step of analyzing one or more characteristics of the one or more entities. On the basis of the determination of the presence of the one or more entities in the sample, or the result of an analysis of the one or more entities, the method may comprise one or more further processing steps, such as data processing steps and/or sample processing steps associated with partitioning and/or isolation of the one or more entities in the sample which was subjected to the assaying procedure.

U.S. Patent Application 2005/275839 A1 discloses a multi-spectral detection and analysis system comprising an observation region, where a targeted sample luminesces when excited by a light source, a multi-channel photodetector array, and a processor that analyzes the spectral signature and compares the spectral signature to known spectral signatures to identify the targeted sample.

U.S. Patent Application 2003/194752 A1 provides methods, reagents and kits for the detection of early sepsis, wherein the methods comprise monitoring biological markers over a period of time, independently deriving for each marker a statistical measure, and applying a decision rule to the combined marker statistics in order to detect early sepsis.

Sven Björnsson et al., Total nucleated cell differential for blood and bone marrow using a single tube in a five-color flow cytometer", Cytometry, Part B, Clinical Cytometry, vol. 74B, no. 2, pages 91-103 discusses the classification of total nucleated cells and platelets in blood or bone marrow by using a flow cytometer.

Many prior art detection methods require the use of expensive laboratory analyzers, which provide graphical/data outputs. These outputs are analyzed by professional staff in order to provide a diagnosis or determination. These kinds of detection methods are expensive, professional labour-intensive and typically centralized at a hospital laboratory. There is thus still a need to provide diagnostic apparatus and methods for quick diagnosis of a patient status.

### SUMMARY OF THE INVENTION

It is an object of the present invention to provide a device, method and systems, which allow for analyzing particles associated with a bodily fluid for the purpose of determining the health state of a patient.

There is thus provided according to an embodiment of the present invention, a system for determination of a medical condition, the system including;
a) a disposable cartridge adapted to receive a volume of a body fluid, the cartridge including a plurality of sections, at least one of the sections adapted to react at least one reactant with the bodily fluid to form a pre-treated sample; and
b) an optics unit including;
   i. at least one excitation illumination adapted to convey radiation to the pre-treated sample;
   ii. at least one multi-spectral emission detector; and
   iii. at least one of a photon counter and an integrator,
wherein the at least one excitation illumination and the at least one multi-spectral emission detector are disposed on the same side of the cartridge; and wherein the optics unit is adapted to detect a plurality of spectrally distinct signals generated by interaction of the radiation and the pre-treated sample in the cartridge, thereby determining the medical condition.

Additionally, according to an embodiment of the present invention, the system further includes a computer, the computer adapted to receive data related to the plurality of spectrally distinct signals and a processor, adapted to process the data and to output at least one output related to the medical condition.

Moreover, according to an embodiment of the present invention, each signal of the plurality of spectrally distinct signals is associated with at least one predetermined biological marker.

Furthermore, according to an embodiment of the present invention, the at least one predetermined biological marker is specific to CD64.

Further, according to an embodiment of the present invention, the at least one predetermined biological marker is specific to CD 163.

Additionally, according to an embodiment of the present invention, the pre-analytical sample processing section includes at least one of the following;
i. an incubator element adapted to incubate the body fluid with at least one antibody;
ii. an incubator element adapted to incubate the body fluid with at least one antigen;
iii. a first mixing element adapted to mix at least one cell type in the body fluid with at least one of a stain and a marker to form at least one of a stained cell type and a marked cell type;
iv. a reacting element adapted to react at least one cell type of the body fluid with the at least one reactant to form the pre-treated sample;
v. a thermal element adapted to heat or cool at least part of the bodily fluid;
vi. a second mixing element adapted to mix at least one cell type in the body fluid with at least one reference material; and
vii. reaction element adapted to form a chemical reaction between the at least one reactant and at least one element of the body fluid.

Additionally, according to an embodiment of the present invention, the sample excitation/interaction section includes a thin metallic lining layer.

Moreover, according to an embodiment of the present invention, the optics unit includes an objective, the objective being adapted to both;
a) pass the radiation to the pre-treated sample; and
b) receive the plurality of spectrally distinct signals.

Furthermore, according to an embodiment of the present invention, the sample excitation/interaction section is disposed perpendicularly to a light path of the objective.

Additionally, according to an embodiment of the present invention, the at least one excitation illumination includes at least one of a laser, and light emitting diode (LED) and an arc lamp.

Furthermore according to an embodiment of the present invention, the pre-treated sample includes particles adapted to flow in an essentially single-file manner past the radiation.

Moreover, according to an embodiment of the present invention, the medical condition is sepsis.

Additionally, according to an embodiment of the present invention, the reader unit includes a plurality of wavelength-specific photomultiplier tubes.

Further, according to an embodiment of the present invention, the particles are white blood cells.

Additionally, according to an embodiment of the present invention, the white blood cells include neutrophils.

Moreover, according to an embodiment of the present invention, the at least one reactant includes multiple fluorescently-tagged antibodies specific for biological markers.

Furthermore, according to an embodiment of the present invention, the system is contained in a portable container of dimension of less than 50 x 30 x 15 cm.

According to another embodiment of the present invention, the system weighs less than 15 kg.

There is thus provided according to another embodiment of the present invention, a method for determining the existence of a health condition in a patient according to claim 10.

Additionally, according to an embodiment of the present invention, steps a) to c) are performed sequentially on a plurality of the pre-treated samples.

According to another embodiment of the present invention, steps a) to c) are performed on 4 to 100 samples in one hour.

Moreover, according to an embodiment of the present invention, each signal of the plurality of spectrally distinct signals is associated with at least one predetermined biological marker.

Additionally, according to an embodiment of the present invention, the at least one predetermined biological marker is specific to CD64.

Furthermore, according to an embodiment of the present invention, the at least one predetermined biological marker is specific to CD163.

According to an embodiment of the present invention, the reacting step includes at least one of;
a) receiving the body fluid directly or indirectly from the patient;
b) performing at least one pre-analytical reaction;
c) exciting the treated sample; and
d) disposing the treated sample in a spent sample disposal section.

Additionally, according to an embodiment of the present invention, the at least one pre-analytical reaction includes at least one of the following;
i. incubating the body fluid with at least one antibody;
ii. incubating the body fluid with at least one antigen;
iii. mixing at least one cell type in the body fluid with at least one of a stain and a marker to form at least one of a stained cell type and a marked cell type;
iv. reacting at least one cell type of the body fluid with the at least one reactant to form the pre-treated sample;
v. thermally treating at least part of the bodily fluid;
vi. mixing at least one cell type in the body fluid with at least one reference material; and
vii. forming a chemical reaction between the at least one reactant and at least one element of the body fluid.

Additionally, according to an embodiment of the present invention, the method further includes performing steps a) to c) on a first sample from the patient.

Furthermore, according to an embodiment of the present invention, the method further includes waiting for a period of time and then repeating steps a) to c) on a further sample.

Moreover, according to an embodiment of the present invention, the bodily fluid includes less than 100 microliters.

Further, according to an embodiment of the present invention, the medical condition is sepsis.

There is thus provided according to a further embodiment of the present invention, a computer software product for determining the existence of a health condition in a patient, the product including a computer-readable medium in which program instructions are stored, which instructions, when read by a computer, cause the computer to;
a) react at least one reactant with a bodily fluid in a multi-sectioned disposable cartridge to form a treated sample;
b) impinge radiation, from an optics unit, on the treated sample in the disposable cartridge thereby generating a plurality of spectrally distinct signals in the direction of the optics unit; and
c) detect a plurality of spectrally distinct signals in the optic unit generated by interaction of the radiation and the pre-treated sample, thereby determining the medical condition.

The invention also provides a device for determining state of health of a patient, including: a disposable cartridge capable of receiving a volume of a bodily fluid, the cartridge including a plurality of chemicals for analytical pretreatment of the fluid; at least one light source, wherein the light source is capable of illuminating particles in the fluid, wherein the particles flow or move past light produced by the light source; and, a reader unit, the reader unit being capable of detecting a plurality of spectrally distinct fluorescent signals generated by interaction of the light and the particles in the cartridge, wherein each signal is associated with at least one predetermined biological marker.

In one aspect of the device, the light source is a laser. In another aspect of the device, the light source is an LED. In another aspect of the device, the light source is an arc lamp.

In another aspect of the device, the particles flow in an essentially single-file manner past the light such that even with one or more particles in the interaction area individual particle information may be obtained.

In another aspect of the device, the particles are moved in an essentially single-file manner past the light such that even with one or more particles in the interaction area individual particle information is/may be obtained.

In another aspect of the device, the bodily fluid is blood or derived from blood.

In another aspect of the device, the disease state is sepsis.

In another aspect of the device, the reader unit includes a plurality of wavelength-specific photomultiplier tubes.

In another aspect of the device, the reader unit includes a plurality of wavelength-specific photomultiplier array elements.

In another aspect of the device, the reader unit includes a plurality of wavelength-specific avalanche photodiodes.

In another aspect of the device, the reader unit includes a plurality of wavelength-specific avalanche photodiode array elements.

In another aspect of the device, the reader unit includes a plurality of wavelength-specific photodetectors.

In another aspect of the device, the reader unit includes a plurality of wavelength-specific photodetector elements.

In another aspect of the device, the at least one predetermined biological marker is specific to CD64.

In still another aspect of the device, the particles are white blood cells.

In another aspect of the device, the particles are beads.

In another aspect of the device, the plurality of chemicals includes multiple fluorescently-tagged antibodies specific for predetermined biological markers.

In another aspect of the device, the reader unit includes 8 photomultiplier tubes or array elements.

In another aspect of the device, there is further including an algorithm for converting fluorescent data to presence of predetermined biological marker information.

In another aspect of the device, the white cells are neutrophils.

In another aspect of the device, a sequence of measurements from the patient is stored or sequences of measurements from the patient are stored.

In another aspect of the device, a sequence of measurements from the patient is analyzed.

The invention includes a method for determining the existence of a predetermined health condition in a patient, including: providing a blood sample; placing a portion of the blood sample in a disposable cartridge, wherein the cartridge includes a plurality of chemicals for treatment of the sample; passing the portion of the blood sample through a region of the cartridge, wherein the region is partially exposed to light generated from at least one light source; recording absorption data from interaction of light with particles in the blood sample; analyzing absorption data; determining absorption characteristics by wavelength; and, presenting to a user of the device the presence of the predetermined disease state in response to the absorption characteristics.

In one aspect of the method, the at least one molecular marker is a plurality of molecular markers. In another aspect of the method, the molecular markers are associated with a plurality of unique fluorescent compounds. In another aspect of the method, the patient is analyzed for the presence of the predetermined disease state on at least two unique occasions. In another aspect of the method, the portion of the blood sample is less than 100 microliters. In another aspect of the method, the predetermined disease is sepsis. In still another aspect of the method, there is further including analyzing the blood sample for left shift of neutrophils.

The combination of cartridge structure, particle flow, illumination and signal processing allows the spectral signature of individual particles to be determined. For example: particles flow in an essentially single-file manner in the blood past light produced by the light source such that even with one or more particles in the interaction area individual particle information is/may be obtained; and, a reader unit, the reader unit being capable of detecting a plurality of spectrally distinct fluorescent signals generated by interaction of the light and the particles in the cartridge, wherein each signal is associated with at least one of the molecular markers.

In one aspect of the device, the fluorescent compounds include FITC, PE, and Syto.

In another aspect of the device, the sequence of stored measurements from a patient is analyzed to determine the health state of the patient.

The system of the present invention offers several advantages over the known art. Firstly, the system comprises an arrangement of optical elements, which allows for construction and use of a "small" flow-cytometer that may be moved to patient bedside for point of care (POC) applications. The system allows for detection and resolution of a plurality of unique fluorescent signals, each associated with a predetermined molecular marker. The invention also includes a unique disposable cartridge that allows for analytical pretreatment of a sample so as to facilitate binding of marker-specific fluorescent probes to their targets in a blood sample. The cartridge also facilitates passage of white blood cells (or other particles) beneath the objective of the cytometer optics to allow for measurement and detection of the presence/absence of predetermined molecular markers. Typical markers include but are not limited to CD64 to identify activated neutrophils, CD163 to identify and differentiate monocytes expressing CD64 from neutrophils expressing CD64, independent of measurements of white blood cell type, number, size and shape. Portions of the system optics may be included in the disposable cartridge, while various methods could be employed to cause sample to pass through a capillary section in the cartridge for interaction with cytometer light. The cytometer may work with laser, LED, or lamp light as per specific application and wavelengths required for testing.

The combination of an epi-optical system, which requires only single sided access to the detection region of the cartridge thus allowing special surface or other treatment of the cartridge significantly reduces the auto-fluorescence of the cartridge material. This reduction is not possible when excitation is introduced on one side of the detection channel and emission is detected on the other side.

The present invention will be more fully understood from the following detailed description of the preferred embodiments thereof, taken together with the drawings.

### BRIEF DESCRIPTION OF THE DRAWINGS

The invention will now be described in connection with certain preferred embodiments with reference to the following illustrative figures so that it may be more fully understood.

With specific reference now to the figures in detail, it is stressed that the particulars shown are by way of example and for purposes of illustrative discussion of the preferred embodiments of the present invention only and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
Fig. 1A a simplified schematic illustration showing a system for rapid determination of a medical condition, in accordance with an embodiment of the present invention;
Fig. 1B is a simplified illustration of a disposable cartridge of the system of Fig. 1A, for rapid determination of a medical condition, in accordance with an embodiment of the present invention;
Fig. 2 a simplified schematic illustration showing another system for rapid determination of a medical condition, in accordance with an embodiment of the present invention;
Fig. 3 is a simplified exploded schematic illustration of an optical unit of the system of Fig. 1 or Fig. 2, in accordance with an embodiment of the present invention;
Fig. 4A is a simplified schematic illustration of an optical arrangement of the optical unit of Fig. 3, in accordance with an embodiment of the present invention;
Fig. 4B is another simplified schematic illustration of optical arrangement of the optical unit of Fig. 3, in accordance with an embodiment of the present invention;
Fig. 5A is a schematic representation of one example of multi-wavelength excitation in the optical unit of Fig. 4A or 4B, in accordance with an embodiment of the present invention;
Fig. 5B shows a graphical output of transmission as a function of wavelength for a dichroic filter of Fig. 4B, employing the multi-wavelength excitation of Fig. 5A, in accordance with an embodiment of the present invention;
Fig. 5C is a schematic representation of part of the optical unit employing multi-wavelength excitation of Fig. 5A and the dichroic filter of Fig. 5B, in accordance with an embodiment of the present invention.
Fig. 6 is a schematic view of a sampling cartridge of the system of Fig. 1 or Fig. 2, in accordance with an embodiment of the present invention;
Fig. 7 shows a schematic view of an alternative embodiment of a disposable cartridge of the system of Fig. 1 or Fig. 2, in accordance with an embodiment of the present invention;
Fig. 8 shows a schematic view of disposable cartridge in flow-cytometer device;
Fig. 9 shows an exemplary view of software output from the system of Fig. 1 or Fig. 2, in accordance with an embodiment of the present invention;
Fig. 10 is a simplified flowchart of a method for rapid determination of a medical condition, in accordance with an embodiment of the present invention;
Fig. 11 is a three-dimensional graph showing the optical output over time of reference beads (RM) relative to a sample from a human patient (PMN), in accordance with an embodiment of the present invention; and
Figs. 12A-12C show graphs of optical outputs over time of the reference beads and the sample from a human patient, in accordance with an embodiment of the present invention.

In all the figures similar reference numerals identify similar parts.

### DETAILED DESCRIPTION OF EMBODIMENTS

In the following description, numerous specific details are set forth in order to provide a thorough understanding of the present invention. It will be apparent, however, to one skilled in the art that the present invention may be practiced without these specific details. In other instances, well-known circuits and control logic have not been shown in detail in order not to unnecessarily obscure the present invention. The following definitions are for aiding in understanding the present invention.

### Definitions

Certain terms are now defined in order to facilitate better understanding of the present invention. Where terms are not defined, it is understood that the generally-accepted meaning in the relevant art may be associated said terms. Thus, "blood", "white cells", and "antibodies" may have their normal meanings as understood in the biological arts, whereas "laser", "beam splitter", and "dichroic filter" may have their normal meanings as understood in the optical arts.

"Cuvette", "cartridge", and "disposable cartridge" may generally refer to an element into which a sample of biological fluid may be entered for the purpose of diagnostic analysis. The element may include a plurality of features including but not limited to pre-analytical treatments, direction-specific flow of sample and optical grating components. A "cartridge" generally is used once and for a single sample derived from blood. A cartridge may be realized as a plurality of cartridges. A cartridge may be made of plastic or any relevant material and may be supplied with a plurality of chemicals in either wet or dry form.

"Flow cytometer" may have its generally understood meaning from the diagnostic arts. A flow cytometer for the present invention may be of "tabletop" size to allow for point-of-care (POC) applications. Additionally, a flow cytometer as per the instant invention generally will have all sample manipulations and flow occur in an associated disposable cartridge and not in the cytometer itself.

"Illness curve" may relate to a chart, plot or the like that shows levels of biological markers or white blood status as a feature of time. As any diagnostic measurement yields information related to the specific health state of a patient at the time when said measurement was made, a medical practitioner may take vital information about the health status of a patient. By taking a plurality of tests over a predetermined period of time, a medical practitioner could compare results to a predetermined illness curve to know in which direction a patient's health is heading. Illness curves are generally defined testing an individual with a known condition over an extended period of time (measured in hours or days) so as to define levels of biological markers over that period of time. These levels will define the illness curve for later use by a physician or the like to determine the time position of a patient with respect to a developing condition.

A "reader unit" or "detector" is an element or unit that may receive and/or process data in the present invention. A reader unit may include a computer and may have additional functionalities including but not limited to data analysis and presentation to a user.

Without being bound by any particular theory, the following discussion is offered to facilitate understanding of the invention. Flow-cytometry is a well-known methodology used throughout the world for analysis of blood and other samples. One of the greatest challenges today is to identify multiple independent, relevant targets to further understand the health condition of a patient. While some illnesses (Streptococcus infection in the throat) may often be diagnosed by a single test, many complex medical conditions may only be accurately defined by the detection, analysis, and study of a plurality of biological markers. As such, a flow-cytometer must for example be capable of defining a plurality of features of white blood cells in order to give valuable information of a potential state of sepsis for a patient. Additionally, a plurality of measurements over a period of time may be required to monitor said features so as to better define where a patient is on an illness curve.

CD64 is a high-affinity and restricted isotype-specificity FcγRI receptor expressed on macrophages, monocytes, neutrophils and eosinophils.

CD163 is a monocyte/macrophage-associated antigen, which has recently be identified as a haemoglobin scavenger receptor.

In the various embodiments disclosed herein, like elements have like reference numerals differing by multiples of 100.

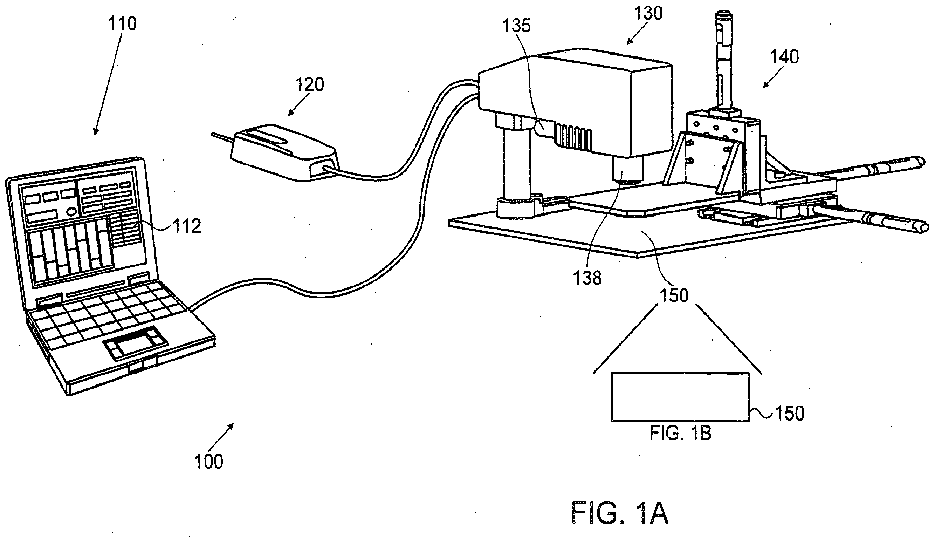

Reference is now made to Fig. 1A, which a simplified schematic illustration showing a system 100 for rapid determination of a medical condition, in accordance with an embodiment of the present invention. System 100 may be considered to be a flow cytometer or micro-flow cytometer, though it differs in its construction, dimensions and operation from the standard prior art flow cytometers, known in the art.

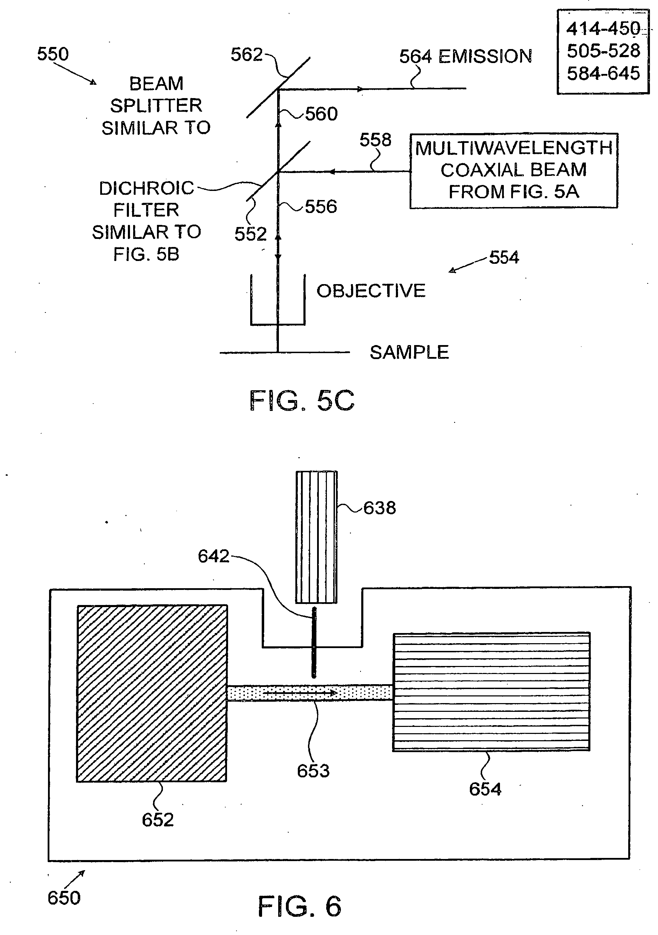

Attention is turned to Fig. 1A, which shows system 100, in accordance with an embodiment of the present invention. A computer 110 is used to both control the system 100, as well as to record results. System 100 includes a power source 120 which may be batteries, wall electricity or any other appropriate source for electrical energy. The power source 120 delivers energy to an optics unit 130, which includes a light source 135, as well as relevant focusing optics (not shown in this figure) for delivery of light through an objective 138 to a disposable sample cartridge (650, Fig. 6 and 150, Fig. 1B), typically placed on an X-Y-Z stage 140. Beneath the optics unit 130 is the X-Y-Z stage 140 that allows for facile exposure of a portion of a disposable cartridge (not shown) for detection of biological markers associated with a blood-based sample (not shown). The X-Y-Z stage may be replaced by a mechanical assembly that precisely locates a cartridge inserted by the user either by automated motion or by fixed mechanical constraints.

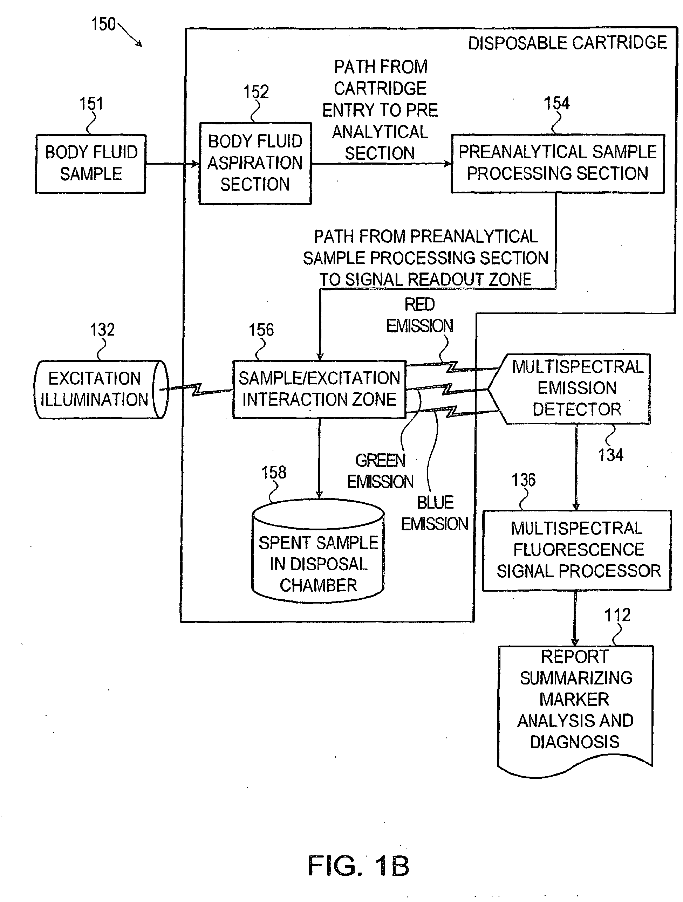

Reference is now made to Fig. 1B, which is a simplified illustration of a disposable cartridge 150 of the system of Fig. 1A, for rapid determination of a medical condition, in accordance with an embodiment of the present invention.

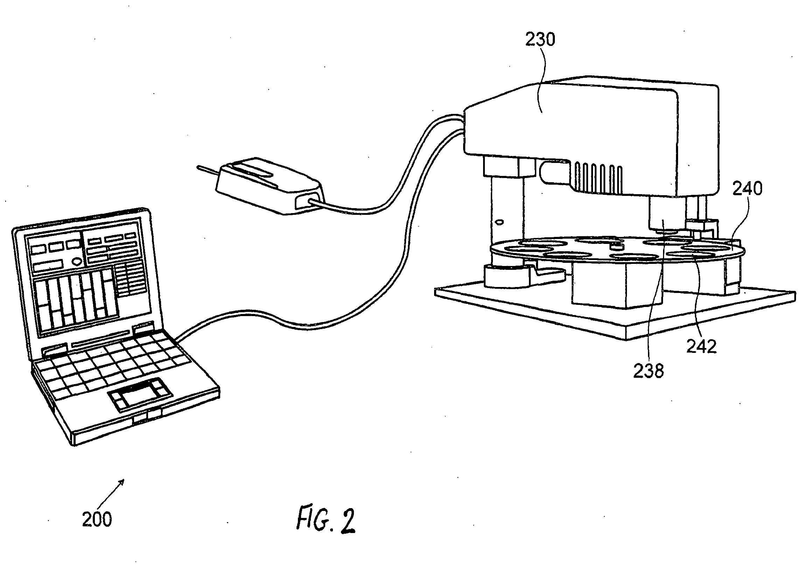

System 100 comprises the disposable cartridge, which may be disposed on an x-y-z stage 140 or, as shown in Fig. 2 a rotating stage 240. The stages are adapted to align the disposable cartridge perpendicularly to a light path of objective 138 of light source 135.

Disposable cartridge 150 is adapted to receive a bodily fluid, such as, but not limited to, blood, urine, serum or plasma. The disposable cartridge is constructed and configured to have several different sections 152, 154, 156 and 158. Section 152 is a body fluid aspiration section, which is adapted to receive the body fluid directly or indirectly from the patient (or animal) and this section acts as a reservoir of the body fluid.

Disposable cartridge 150 comprises fluid conveying means between the sections, such as, but not limited to, air pressure, liquid pressure, mechanical means and combinations thereof. Body fluid aspiration section 152 is adapted to convey a predetermined quantity of the body fluid (a body fluid sample 151) to a pre-analytical sample processing section 154.

In pre-analytical sample processing section 154, at least one preparatory step is performed on the body fluid such as, but not limited to:
a) incubation with at least one antibody;
b) incubation with at least one antigen;
c) staining of at least one cell type in the body fluid;
d) enzymatic lysing of at least one cell type of the body fluid;
e) osmotic lysing of at least one cell type of the body fluid;
f) heat or cool at least part of the bodily fluid;
g) addition of reference material to the bodily fluid; and
h) chemical reaction with at least one element of the body fluid.

The pre-treated sample of bodily fluid is then conveyed from pre-analytical sample processing section 154 to a sample excitation/interaction zone or section 156. This pre-treated sample may be conveyed continuously or in a batch mode to sample excitation/interaction section 156.

Sample excitation/interaction section 156 may be disposed or brought into position by the x-y-z stage 140 or, as shown in Fig. 2 a rotating stage 240 to sit in the light path of an excitation illumination 132, which is an integral part of optics unit 130. The excitation illumination is constructed and configured to pass radiation, such as coherent or incoherent radiation in or outside the visible range into sample excitation/interaction section 156 containing the pre-treated sample. Resultant emission or emissions from the pre-treated sample is passed from sample excitation/interaction section 156 to a multi-spectral emission detector 134, housed in optics unit 130. Sample excitation/interaction section 156 is constructed and configured to minimize auto-fluorescence from the disposable sample cartridge material.

Cartridge 150 is constructed out of a plastic material. Sample excitation/interaction section 156 comprises a thin metallic lining layer 157 (not shown) of around 0.1-2 microns. The metal may be aluminum, gold, silver, nickel, copper or any other suitable conductive metal or alloy. The thin metallic lining layer is constructed and configured to prevent the excitation illumination from entering the plastic material of construction of the cartridge.

Multi-spectral emission detector 134 receives the emission from the pre-treated sample and is adapted to sample the spectral emission in spectral bands. In some cases these bands are non-overlapping bands. Multi-spectral emission detector 134 is adapted to pass data representing the spectral bands to multi-spectral fluorescence signal processor 136.

Multi-spectral fluorescence signal processor 136 may comprise two or more sub-elements (not shown) including:
a) a photon counter 131, as part of the optics unit 130;
b) a software element 111 (in computer 110) for analyzing the photon counter output; and
c) other detecting elements (not shown) for measuring other optical outputs of multi-spectral emission detector 134.

The cartridge further comprises a spent sample disposal section 158, adapted to receive a sample from the sample excitation/interaction section.

The system further comprises computer 110, the computer is adapted to receive data related to the plurality of spectrally distinct signals and a processor, adapted to process said data and to output at least one output related to said medical condition. One type of output provided is a visual output which is outputted onto a screen 112 of the computer.

According to some embodiments of the present invention, system 100 is designed to be portable. In some cases, the system, including computer 110, is contained in a portable container (not shown), such as a box or case having dimensions of less than 50 x 30x 15 cm. According to some embodiments, the dimensions of the system are 40 x 25 x 10 cm, while the disposable cartridge dimensions are 8.5 x 5.4 x 0.6 cm. The system typically weighs less than 15 kg. According to some embodiments, it weighs less than 10 kg.

Reference is now made to Fig. 2, which a simplified schematic illustration showing another system 200 for rapid determination of a medical condition, in accordance with an embodiment of the present invention. System 200 comprises a rotating stage 240 for presenting multiple sample cartridges, seen as round depressions 242 on stage 240, beneath an objective 238 of an optics unit 230. One advantage of the systems of the present invention becomes obvious in Fig. 2. Since sample preparation and liquid flow occurs specifically in a cartridge, multiple samples may be prepared simultaneously, with each sample sequentially analyzed by the diagnostic unit. This feature is an enormous advantage over the present art, in which sample necessarily flows through a single flow-cell in a cytometer and as such one sample may be processed at a time.

As processing times may be twenty minutes or longer, the effective testing rate of a prior art cytometer is three samples per hour. In the instant invention, numerous samples may be prepared (pre-analytical treatment) and then sequentially exposed to light from the optics unit 230 to record fluorescent signals. According to some embodiments, 4-50 samples can be processed in one hour. According to some further embodiments, up to one hundred samples can be analyzed in one hour. According to some further embodiments, up to five hundred samples can be analyzed in one hour.

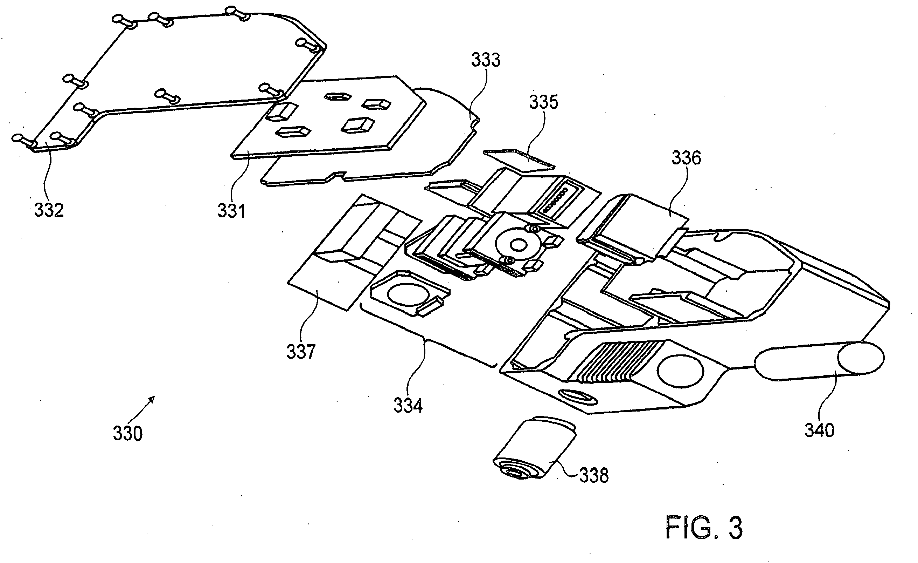

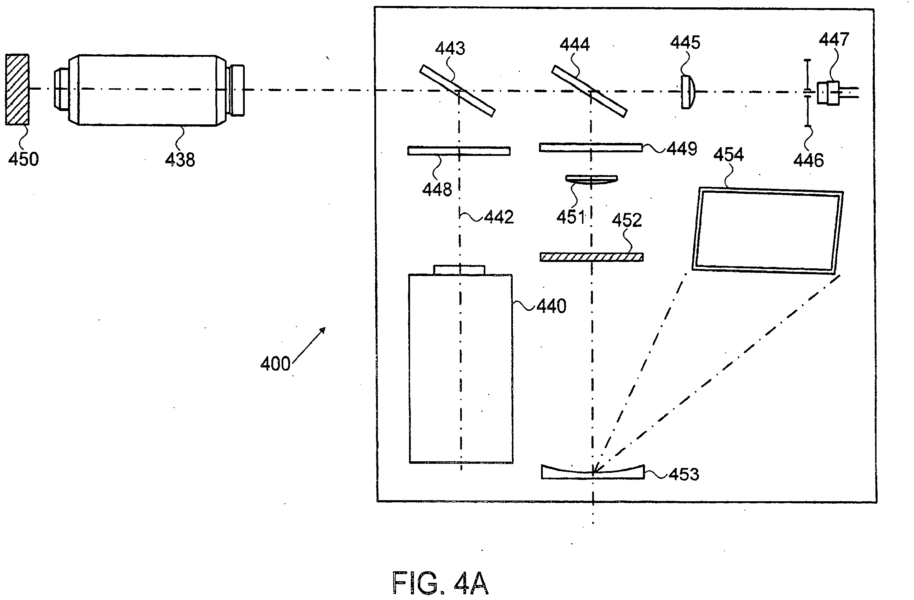

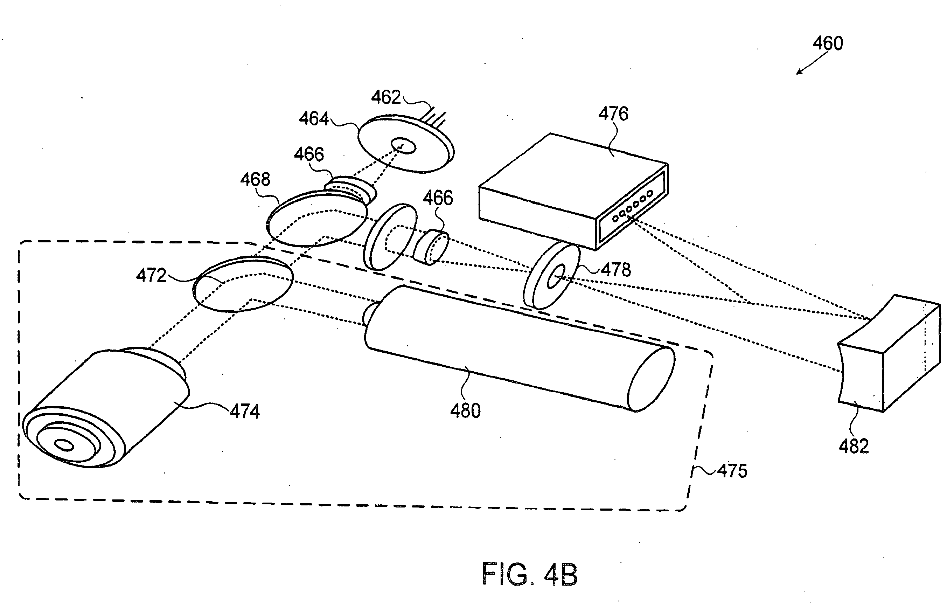

Reference is now made to Fig. 3, which is a simplified exploded schematic illustration of an optical unit 330 of the system 100 of Fig. 1 or system 200 of Fig. 2, in accordance with an embodiment of the present invention. The optical apparatus comprises a main PCB 331, sandwiched between an external cover 332 and a polychromator cover 333. Optical elements 334 (further defined in Fig. 4), an auto-focus feedback PCB 335, a grating assay 336, and a power-supply PCB 337 are present, in addition to a laser 340 and an optical objective 338. The combination of various optical elements in the optical unit 330 allows for powerful detection and resolution of multiple fluorescent signals in a relatively small and compact unit. This size feature allows for the potential for POC application of flow-cytometry.

Reference is now made to Fig. 4A, which is a simplified schematic illustration of an optical arrangement 400 of the optical unit of Fig. 3, in accordance with an embodiment of the present invention. A laser 440 or other appropriate light source provides a light beam 442, which may be directed towards a plurality of optical elements, including a dichroic filter 443, a beam splitter 444, a focusing lens 445, a pinhole 446 and a silicon reader unit 447, for recording a signal from a beam 442 directed through the objective 438 towards a sample 450 and returned to the optical unit. Additional optical elements may include an optional attenuator 448, a high-pass filter 449, a focusing lens 451, a slit 452, a concave grating 453, and a PMT array 454. This arrangement of elements, representing an embodiment of the present invention, allows for generation of excitation light, focusing it on a sample, collecting reflected and emitted light signal resulting from the interaction of the excitation light and fluorophores in the sample and recording said returned light so as to determine fluorescence of sample in response to light illumination from laser 440.

With respect to Fig. 4A, the laser illumination 442 is reflected by the dichroic filter 443 through the objective 438 and focused on the channel containing the flowing particles 458. This illumination excites the fluorophores attached to the protein markers that are bound to the cells. The resulting fluorescent illumination is collected by the objective 438 and because of the longer wavelength of this emission passes through the dichroic filter 443 and is reflected by the beam splitter 444 through the high pass filter 449. The high pass filter blocks any reflected laser illumination. The focusing lens 451 focuses the multi-wavelength emission illumination on the slit 452. The concave grating 453 images the slit at multiple wavelengths on the elements of the PMT array 454. This completes the process of creating a multispectral detection of the fluorescent emission. While most of the illumination collected by the objective is reflected by the beam splitter 444 a small fraction is allowed to pass through and is focused by focusing lens 445 through a pinhole 446 on the silicon reader unit 447, which may be a single photodiode or a focal plane array such as CCD sensor. During the focusing operation best focus is achieved when the signal on this reader unit 447 is maximized. When this signal is maximized, the intensity of the signal on the PMT array 454 is also maximized.

Reference is now made to Fig. 4B, which is a simplified illustration of the optical arrangement 460 of optical unit 300 of Fig. 3, in accordance with an embodiment of the present invention.

A laser 480 or other appropriate light source provides a light beam 484, which may be directed towards a plurality of optical elements, including a dichroic filter 472, a beam splitter 468, a focusing lens 466, a pinhole 464 and a silicon reader unit 462, for recording a signal from a beam 442 directed through the objective 438 towards a sample and returned to the optical unit. Additional optical elements may include an optional attenuator, a high-pass filter 470, a focusing lens 466, a slit 478, a concave grating 482, and a PMT array 476. This arrangement of elements, representing an embodiment of the present invention, allows for generation of excitation light, focusing it on a sample, collecting reflected and emitted light signal resulting from the interaction of the excitation light and fluorophores in the sample and recording said returned light so as to determine fluorescence of sample in response to light illumination from laser 440.

With respect to Fig. 4B, as in Fig 4A, the laser illumination is reflected by the dichroic filter 472 through the objective 476 and focused on the channel containing the flowing particles. This illumination excites the fluorophores attached to the protein markers that are bound to the cells. The resulting fluorescent illumination is collected by the objective 476 and because of the longer wavelength of this emission passes through the dichroic filter 472 and is reflected by the beam splitter 468 through the high pass filter 470. The high pass filter 470 blocks any reflected laser illumination. The focusing lens 466 focuses the multi-wavelength emission illumination on the slit 478. The concave grating 482 images the slit at multiple wavelengths on the elements of the PMT array 476. This completes the process of creating a multispectral detection of the fluorescent emission. While most of the illumination collected by the objective 476 is reflected by the beam splitter 468 a small fraction is allowed to pass through and is focused through a pinhole 464 on the silicon reader unit 462. During the focusing operation best focus is achieved when the signal on this reader unit is maximized. When this signal is maximized, the intensity of the signal on the PMT array 476 is also maximized.

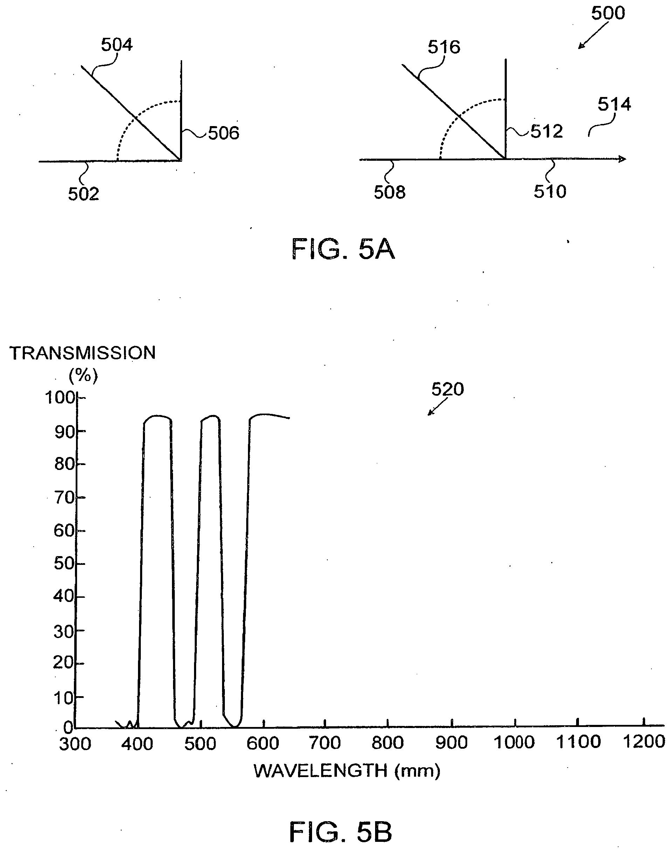

Reference is now made to Fig. 5A, which is a schematic representation 500 of one example of multi-wavelength excitation in the optical unit of Fig. 4A or 4B, in accordance with an embodiment of the present invention. Figs. 5A-5C show an extension of the optical configuration in Figs. 4A and 4B, to allow multiple excitation wavelengths. Fig. 5A shows the configuration for combining multiple lasers of different wavelengths to yield a single coaxial beam 514 containing all of the wavelengths. Two different wavelengths, such as 502 green and 506 red, may be combined using a dichroic mirror 504. One of the beams, red 506 is reflected by the dichroic mirror, while the second beam, green 502 passes through the dichroic mirror to yield a single beam 508, yellow, containing both wavelengths. This combined wavelength beam is now used as one of the inputs to a second dichroic mirror 510 with the third wavelength 512 being reflected by the second dichroic mirror to yield a single coaxial beam containing all three wavelengths.

Reference is now made to Fig. 5B, which shows a graphical output 520 of transmission as a function of wavelength for a dichroic filter of Fig. 4B, employing the multi-wavelength excitation of Fig. 5A, in accordance with an embodiment of the present invention. A multiband dichroic mirror (not shown) similar, or identical to, mirror 552 of Fig. 5C is used to illuminate the sample through an objective 554 (Fig. 5C), while allowing the resulting emission to pass through dichroic mirror 552 at all wavelengths, except those of multibeam excitation 514 (Fig. 5A). In this way the same epi-configuration used with a single wavelength can, in fact, be used with appropriate changes to dichroic mirror 552 and the addition of multiple lasers 502, 506, 512 to provide multi-wavelength excitation, while maintaining virtually all of the detection wavelengths of a single excitation system.

Turning to Fig. 5C, a schematic representation of part 550 of the optical unit is seen, employing multi-wavelength excitation of Fig. 5A and the dichroic filter of Fig. 5B, in accordance with an embodiment of the present invention. Part 550 may, in some cases, replace subsystem 475 (Fig. 4B).

**Table 1 shows representative values for representative components for use in the present invention.**

| | | |
|---|---|---|
| Laser Wavelength | 405nm | 488nm |
| Laser Power | 50mW | 20mW |
| Sensing Spectral Range | 200nm | 200nm |
| Spectral Resolution | 25nm | 25nm |
| Number of Detectors | 8 | 8 |
| Collecting Optics | Microscope Objective N.A.>0.4, W.D. ≈ 6mm | Microscope Objective N.A.>0.4, W.D. ≈ 6mm |
| Detector Type | S.S. PMT 8 ch | S.S. PMT 8 ch |

While much of the previous discussion has focussed on the optical elements of some embodiments of the present invention, one of the key components of the diagnostic system herewith presented is a disposable sample cartridge.

Reference is now made to Fig. 6, which is a schematic view of a sampling cartridge 650 of the system of Fig. 1 or Fig. 2, in accordance with an embodiment of the present invention. The cartridge 650 includes a pre-analytical component 652 into which a sample (not shown) may be introduced. The sample will generally be blood, either whole or a component (serum, etc.) thereof. Other liquid samples may additionally or alternatively be employed. In the pre-analytical component 652, the sample is allowed to interact with chemicals pre-packaged into component 652. The interaction may be either passive or include active mixing. The chemicals included in the analytical component 652 may be either wet or dry, and generally include antibodies associated with fluorescent probes. Antibodies are pre-selected for their ability to bind with predetermined biological markers or the like. In a typical experiment, a predetermined volume (generally less than 50 microliters) of blood is introduced into the pre-analytical component 652 of a disposable cartridge 650. The sample is actively mixed with chemical reagents present in the pre-analytical component 652 for a predetermined period of time, generally less than ten minutes. The sample is then moved through a capillary region 653 by means to be discussed, where it is exposed to a light beam 642 delivered from an objective 638. Direction of sample flow is as shown by the arrow in the capillary region 653.

The capillary region 653 is designed to allow flow of particles in a single-file past the light beam 642. Such an arrangement allows both for counting the number of particles as well as individual interrogation of particles to determine the presence of biological markers (via their associated fluorescent tags) on each particle. Such a physical arrangement allows for detection of one or more biological markers (independent of particle-specific properties such as size, shape, and number) on each particle.

Finally, there is a collection component 654 which receives sample after exposure to light beam 642. This is a waste region and allows for a completely self-contained disposable for sample preparation, analysis and waste collection. It is noted that the disposable cartridge may be of any relevant shape and is shown as it is in Fig. 6 for ease of understanding of its components and functionality.

As mentioned above, the sample, after pre-analytical treatment to allow for binding of fluorescent tag to cells/particles, must flow under a light beam 642, produced by an optical unit (not shown). The flow is generally "single file" so as to allow for accurate determination of cell-specific markers on each analyzed cell. Methods to induce flow include but are not limited to electrical stimulation, chemical induction, and vacuum pull. In an electrical stimulation system, charge is applied across the capillary region 653 so as to induce charged particles to move from the pre-analytical component 652 towards the collection component 654. The charge could be supplied by the cytometer in which the disposable cartridge 650 is placed or from an external source.

Alternatively, the capillary region may include chemical features (hydrophilic/hydrophobic; positive/negative charge) to encourage sample to move from left to right as shown in Fig. 6. Alternatively, a vacuum from the collection component 654 could be applied to pull sample from the pre-analytical component 652 through the capillary region 653. Other methods may be employed to get liquid sample to move underneath the light beam 642 for analysis.

As described herein, the optics and sample handling have been handled separately. Such an arrangement is not mandatory, as some of the optical features needed for proper sample analysis may be included in a disposable cartridge.

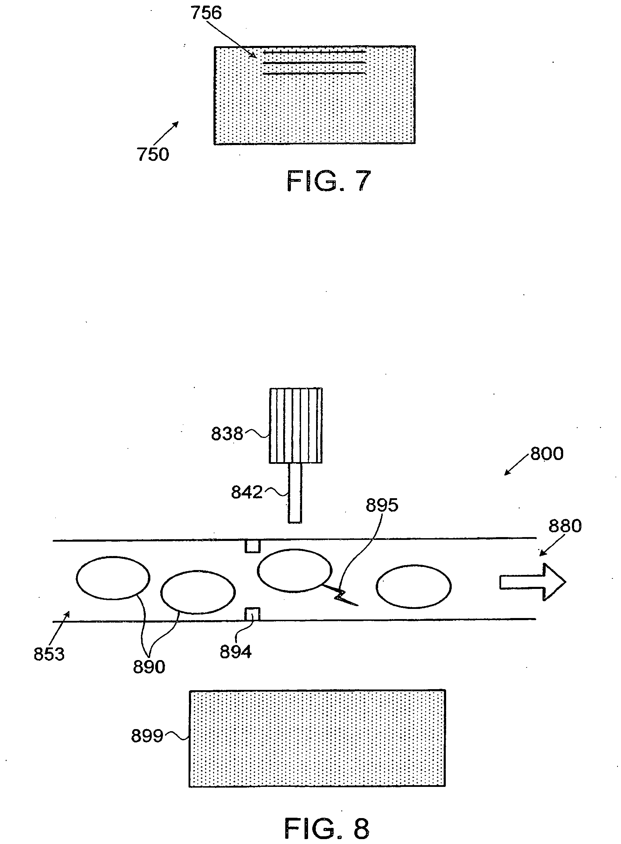

Reference is now made to Fig. 7, which shows a schematic view of an alternative embodiment of a disposable cartridge 750 of the system of Fig. 1 or Fig. 2, in accordance with an embodiment of the present invention. Optical gratings 756 are included in the cartridge 750. Such an arrangement may allow for further miniaturization and simplification of the optical unit (not shown).

Reference is now made to Fig. 8, which shows a schematic view of disposable cartridge 800 in flow-cytometer device. Attention is currently turned to Fig. 8 which shows an expanded view of a capillary region 853. In the capillary region 853, particles flow in the direction as suggested by the arrow 880. Particles 890 flow past an objective 838 that shines light 842 through the capillary 853. Flow restriction elements 894 may be present in the capillary region 853 so as to encourage particles 890 to move past the light 842 in a nearly single-file manner. Passage of multiple particles together may be resolved through processing software.

A molecular marker 895 on a particle 890 may be illuminated by light 842 and its fluorescence will be captured by a proximate photomultiplier tube 899. The photomultiplier tube 899 may distinguish the wavelength of the fluorescence and thus which biological marker 895 is present on particle 890. Thus, the systems of the present invention may determine which biological markers are present on particles 890, which are detected in the systems of the present invention. A photomultiplier tube 899 may have a plurality of tubes or an array of elements for fine wavelength discrimination and alternatively may be replaced with film, CCD or other appropriate light-receiving reader unit. It should be understood that Fig. 8 shows one embodiment of the configuration of system 100 (Fig. 1) in a transmissive configuration, wherein detector (photomultiplier tube 899) is disposed on an opposing side of the cartridge 800 to objective 838. This is in contrast to the setup in Fig. 1, 2, 4A and 4B.

The systems of the present invention comprise controller software which are adapted to run a diagnostic process. It is understood that the controller software may be an integral part of the flow-cytometer or alternatively be installed on an associated computing device (see Figs 1 & 2), which may include, but not be limited to, a laptop computer, iPod, iPad, cell phone or mainframe computer.

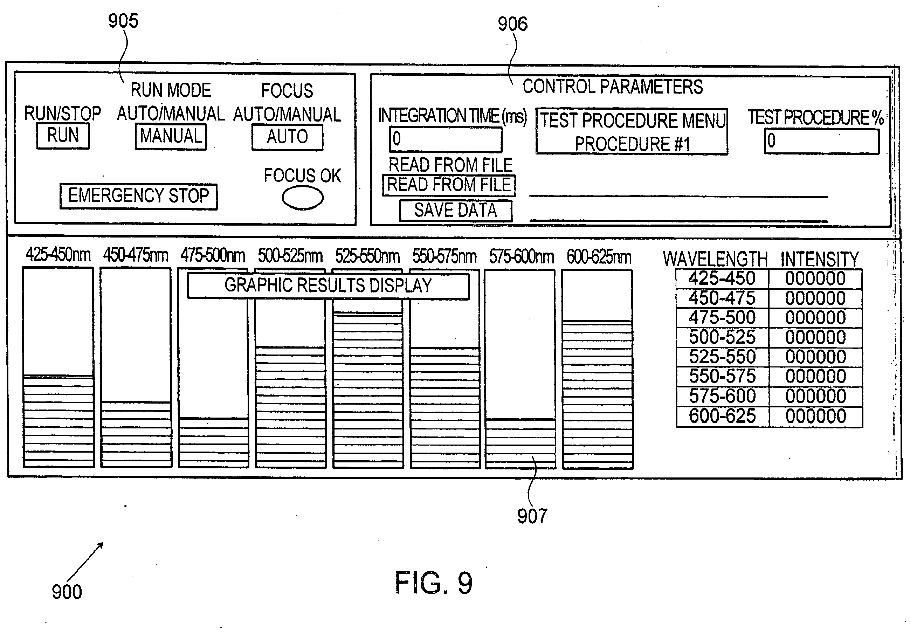

Reference is now made to Fig. 9, which shows an exemplary view of software output from the system of Fig. 1 or Fig. 2, in accordance with an embodiment of the present invention. In Fig. 9, an exemplary screen shot 900 is shown. Specifically, the software as depicted in Fig. 9 allows both for controlling the flow-cytometer, as well as for collecting/displaying output data.

In one embodiment, screen shot 900, is divided into three regions. The top left region 905 includes controls for running/stopping/calibrating the system or flow cytometer (100, Fig. 1). The top right region 906 allows for file management/storage. The bottom region 907 concerns data output, with data being divided by fluorescence wavelength regions, for example, as shown.

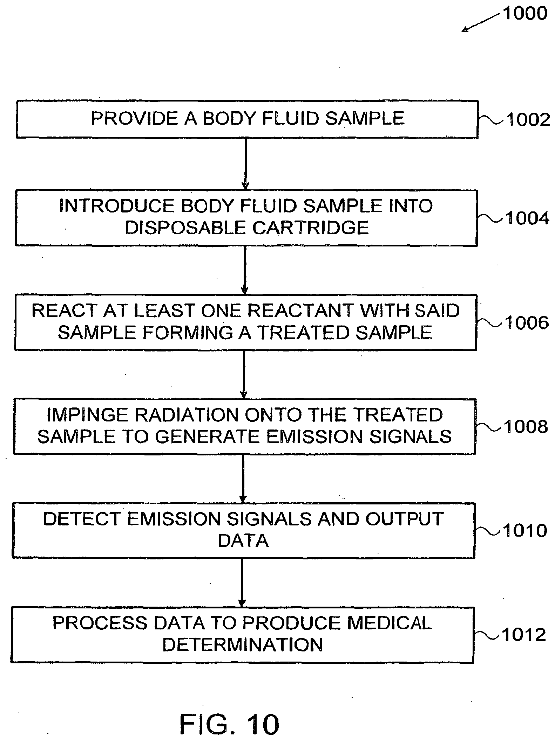

Reference is now made to Fig. 10, which is a simplified flowchart 1000 of a method for rapid determination of a medical condition, in accordance with an embodiment of the present invention. It is to be understood that the method described herein depicts one non-limiting embodiment of the present invention for determining the health state of a patient. Further embodiments are also construed to be part of the present invention.

In a body fluid provision step 1002, a body fluid, such as blood, urine, serum or plasma is provided from a human or animal patient. Typically, the sample is fresh, but may also be a stored, refrigerated or frozen-thawed sample. The fluid is typically liquid and at a temperature of 4-37 °C.

In a body fluid introduction step 1004, part or all of the body fluid sample 151 (Fig. 1B) is introduced into disposable cartridge (150, Fig. 1B, or 650, Fig. 6 or 750 Fig. 7 or 800, Fig. 8). According to some embodiments, the fluid sample is introduced into body fluid aspiration section 152 (Fig. 1B).

In a reacting step 1006, the fluid sample is reacted with at least one reactant in the cartridge forming a treated sample. According to some embodiments, this step is performed in pre-analytical sample processing section 154 (Fig. 1B) as described in detail hereinabove.

In an impinging step 1008, radiation is impinged on the treated sample, such as, but not limited to, in sample excitation/interaction section 156, thereby generating a plurality of spectrally distinct signals in the direction of optics unit 130 (Fig. 1, see description hereinabove).

In a spectral emissions detection step 1010, a plurality of spectrally distinct signals is detected by multiple emission detector 134 (Fig. 1B). The detector outputs data.

Thereafter, in a data processing step 1012, the outputted data is processed by signal processor 136 and/or by computer 110 to provide an output indicative of a medical condition.

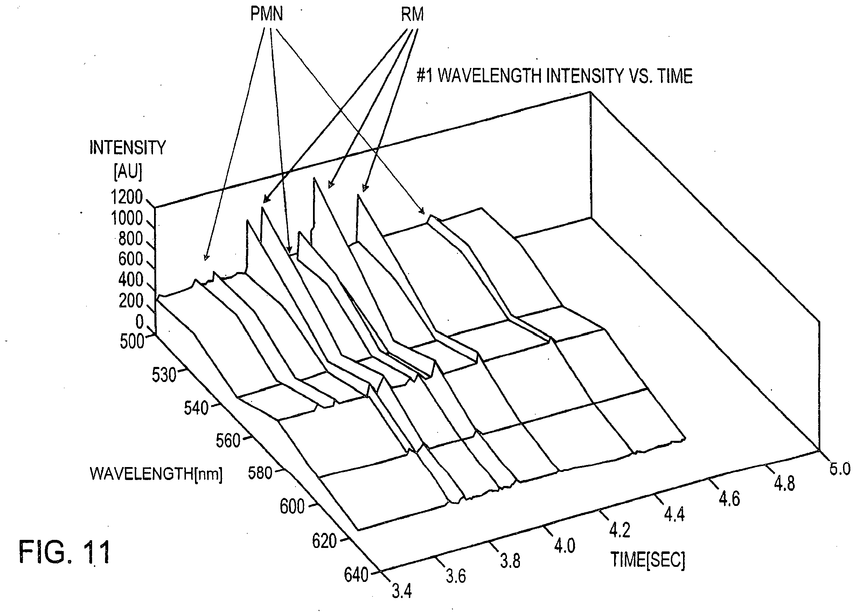

Fig. 11 shows a three-dimensional graph showing the optical output over time of reference beads (RM) relative to a sample from a human patient (PMN), in accordance with an embodiment of the present invention. The emission amplitude in the six bands, 500-525 nm, 525-550 nm, 550-575 nm, 575-600 nm, 600-625 nm and 625 to 650 nm is displayed in the graph for each sample time. Different fluorophores have different emission spectra. It can be appreciated that both spectral content or shape and amplitude at individual wavelengths are significantly different for neutrophils stained with Acridine Orange (AO) and reference beads (RM) containing a bright broad spectrum fluorophore. The peak of the AO emission is in the 525-550 nm band, while that of RM is in the 500-525 nm band and is of a significantly greater amplitude than AO in any band.

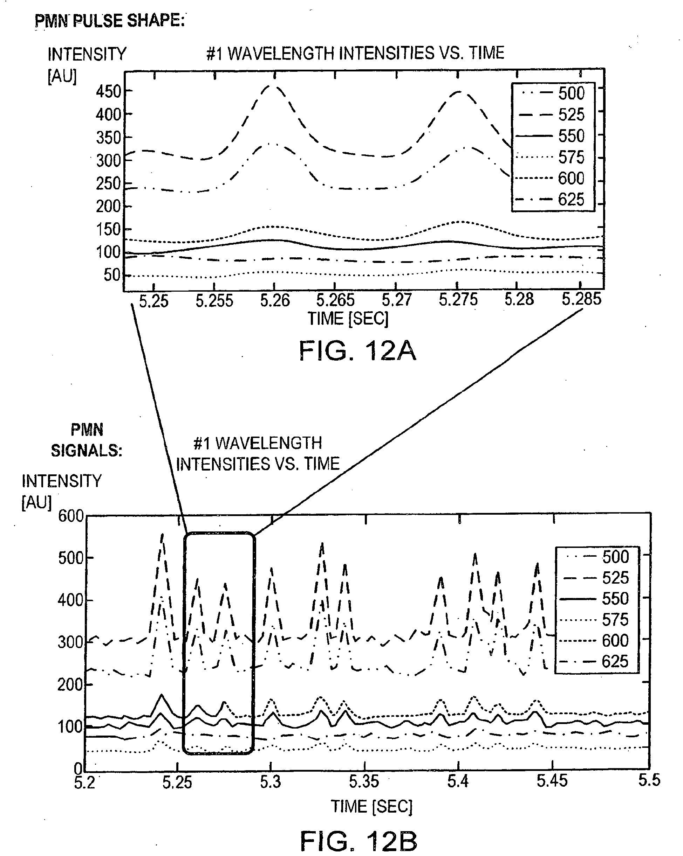

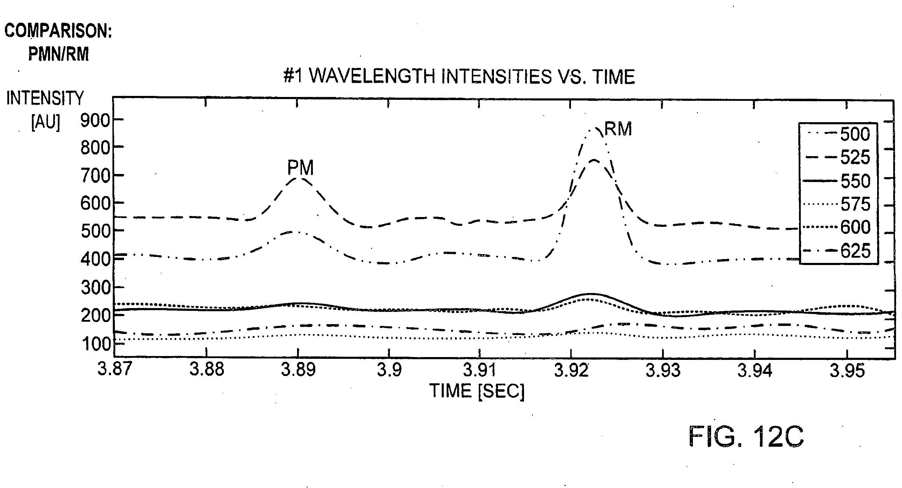

Turning to Figs. 12A-12C, there can be seen graphs of optical outputs over time of the reference beads and the sample from a human patient, in accordance with an embodiment of the present invention. In these two-dimensional figures, the traces from each of the bands are overlaid on the same graph. Fig. 12A shows the boxed pulses from neutrophils in Fig. 12B. It is clear from these graphs that the amplitude in the 525-550 nm channel exceeds the amplitude in the 500-525 nm channel, which is the characteristic of AO. Fig. 12C shows a comparison of the AO stained neutrophil emission spectrum to that of the RM emission spectrum. The relative amplitude of the spectrum in the 500-525 nm band to that of the amplitude in the 525-550 nm band clearly distinguishes the two fluorophores. In addition, the maximum amplitude of the RM emission is significantly greater that that of AO.

The systems of the present invention, as described and shown herein provide uses, such as, but not limited to, at least one of the four following scenarios:
a) When multiple pieces of information, such as biological markers and white cell state are required in order to make an accurate diagnostic determination;
b) When multiple sequential measurements must be made in order to determine the position of a patient on an illness curve;
c) When white cell and similar data are needed quickly and in a POC environment; and
d) When fluorescent signals overlap in wavelength and there is need to determine relative contribution of each signal for a given wavelength range.

The instant invention includes software and algorithms for proper data analysis and conversion of raw fluorescence data into actual concentrations of relative biological markers.

Other suitable operations or sets of operations may be used in accordance with some embodiments. Some operations or sets of operations may be repeated, for example, substantially continuously, for a pre-defined number of iterations, or until one or more conditions are met. In some embodiments, some operations may be performed in parallel, in sequence, or in other suitable orders of execution.

Discussions herein utilizing terms such as, for example, "processing," "computing," "calculating," "determining," "establishing", "analyzing", "checking", or the like, may refer to operation(s) and/or process(es) of a computer, a computing platform, a computing system, or other electronic computing device, that manipulate and/or transform data represented as physical (e.g., electronic) quantities within the computer's registers and/or memories into other data similarly represented as physical quantities within the computer's registers and/or memories or other information storage medium that may store instructions to perform operations and/or processes.

Some embodiments may take the form of an entirely hardware embodiment, an entirely software embodiment, or an embodiment including both hardware and software elements. Some embodiments may be implemented in software, which includes but is not limited to firmware, resident software, microcode, or the like.

Some embodiments may utilize client/server architecture, publisher/subscriber architecture, fully centralized architecture, partially centralized architecture, fully distributed architecture, partially distributed architecture, scalable Peer to Peer (P2P) architecture, or other suitable architectures or combinations thereof.

Some embodiments may take the form of a computer program product accessible from a computer-usable or computer-readable medium providing program code for use by or in connection with a computer or any instruction execution system. For example, a computer-usable or computer-readable medium may be or may include any apparatus that can contain, store, communicate, propagate, or transport the program for use by or in connection with the instruction execution system, apparatus, or device.

In some embodiments, the medium may be or may include an electronic, magnetic, optical, electromagnetic, InfraRed (IR), or semiconductor system (or apparatus or device) or a propagation medium. Some demonstrative examples of a computer-readable medium may include a semiconductor or solid state memory, magnetic tape, a removable computer diskette, a Random Access Memory (RAM), a Read-Only Memory (ROM), a rigid magnetic disk, an optical disk, or the like. Some demonstrative examples of optical disks include Compact Disk-Read-Only Memory (CD-ROM), Compact Disk-Read/Write (CD-R/W), DVD, or the like.

In some embodiments, a data processing system suitable for storing and/or executing program code may include at least one processor coupled directly or indirectly to memory elements, for example, through a system bus. The memory elements may include, for example, local memory employed during actual execution of the program code, bulk storage, and cache memories which may provide temporary storage of at least some program code in order to reduce the number of times code must be retrieved from bulk storage during execution.

In some embodiments, input/output or I/O devices (including but not limited to keyboards, displays, pointing devices, etc.) may be coupled to the system either directly or through intervening I/O controllers. In some embodiments, network adapters may be coupled to the system to enable the data processing system to become coupled to other data processing systems or remote printers or storage devices, for example, through intervening private or public networks. In some embodiments, modems, cable modems and Ethernet cards are demonstrative examples of types of network adapters. Other suitable components may be used.

Some embodiments may be implemented by software, by hardware, or by any combination of software and/or hardware as may be suitable for specific applications or in accordance with specific design requirements. Some embodiments may include units and/or sub-units, which may be separate of each other or combined together, in whole or in part, and may be implemented using specific, multi-purpose or general processors or controllers. Some embodiments may include buffers, registers, stacks, storage units and/or memory units, for temporary or long-term storage of data or in order to facilitate the operation of particular implementations.

Some embodiments may be implemented, for example, using a machine-readable medium or article which may store an instruction or a set of instructions that, if executed by a machine, cause the machine to perform a method and/or operations described herein. Such machine may include, for example, any suitable processing platform, computing platform, computing device, processing device, electronic device, electronic system, computing system, processing system, computer, processor, or the like, and may be implemented using any suitable combination of hardware and/or software. The machine-readable medium or article may include, for example, any suitable type of memory unit, memory device, memory article, memory medium, storage device, storage article, storage medium and/or storage unit; for example, memory, removable or non-removable media, erasable or non-erasable media, writeable or re-writeable media, digital or analog media, hard disk drive, floppy disk, Compact Disk Read Only Memory (CD-ROM), Compact Disk Recordable (CD-R), Compact Disk Re-Writeable (CD-RW), optical disk, magnetic media, various types of Digital Versatile Disks (DVDs), a tape, a cassette, or the like. The instructions may include any suitable type of code, for example, source code, compiled code, interpreted code, executable code, static code, dynamic code, or the like, and may be implemented using any suitable high-level, low-level, object-oriented, visual, compiled and/or interpreted programming language, e.g., C, C++, Java, BASIC, Pascal, Fortran, Cobol, assembly language, machine code, or the like.

Functions, operations, components and/or features described herein with reference to one or more embodiments, may be combined with, or may be utilized in combination with, one or more other functions, operations, components and/or features described herein with reference to one or more other embodiments, or vice versa.

Any combination of one or more computer usable or computer readable medium(s) may be utilized. The computer-usable or computer-readable medium may be, for example but not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CDROM), an optical storage device, a transmission media such as those supporting the Internet or an intranet, or a magnetic storage device. Note that the computer-usable or computer-readable medium could even be paper or another suitable medium upon which the program is printed, as the program can be electronically captured, via, for instance, optical scanning of the paper or other medium, then compiled, interpreted, or otherwise processed in a suitable manner, if necessary, and then stored in a computer memory. In the context of this document, a computer-usable or computer-readable medium may be any medium that can contain, store, communicate, propagate, or transport the program for use by or in connection with the instruction execution system, apparatus, or device. The computer-usable medium may include a propagated data signal with the computer-usable program code embodied therewith, either in baseband or as part of a carrier wave. The computer usable program code may be transmitted using any appropriate medium, including but not limited to wireless, wireline, optical fiber cable, RF, etc.

Computer program code for carrying out operations of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

The present invention is described herein with reference to flow chart illustrations and/or block diagrams of methods, apparatus (systems) and computer program products according to embodiments of the invention. It will be understood that each block of the flow chart illustrations and/or block diagrams, and combinations of blocks in the flow chart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer-readable medium that can direct a computer or other programmable data processing apparatus to function in a particular manner, such that the instructions stored in the computer-readable medium produce an article of manufacture including instruction means which implement the function/act specified in the flow charts and/or block diagram block or blocks.

The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the flow charts and/or block diagram block or blocks.

The flow charts and block diagrams in the Figures illustrate the architecture, functionality, and operation of possible implementations of systems, methods and computer program products according to various embodiments of the present invention. In this regard, each block in the flow charts or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow chart illustrations, and combinations of blocks in the block diagrams and/or flow chart illustrations, can be implemented by special purpose hardware-based systems that perform the specified functions or acts, or combinations of special purpose hardware and computer instructions.

Although the embodiments described above mainly address assessing test coverage of software code that subsequently executes on a suitable processor, the methods and systems described herein can also be used for assessing test coverage of firmware code. The firmware code may be written in any suitable language, such as in C. In the context of the present patent application and in the claims, such code is also regarded as a sort of software code.

## Claims

1. A micro-flow cytometer system (100, 200) for determination of a medical condition, the system comprising:
a) a multi-sectioned disposable cartridge (150) adapted to have manipulation and flow of samples occur therein, said cartridge adapted to receive a volume of a body fluid, said cartridge comprising a plurality of sections comprising:
i. a body fluid aspiration section (152) adapted to receive the body fluid directly or indirectly from a patient;
ii. a pre-analytical sample processing section (154) adapted to react at least one reactant with said bodily fluid to form a pre-treated sample;
iii. a sample excitation/interaction section (156);
iv. fluid conveying means for conveying said pre-treated sample from said pre-analytical sample processing section to said sample excitation/interaction section; and
v. a spent sample disposal section (158);
b) an optics unit (130) comprising:
i. an excitation illumination (132) adapted to convey radiation via a first pathway to said pre-treated sample via a pathway;
ii. an multi-spectral emission detector (134) adapted to sample the spectral emission in spectral bands; and
iii. an of a photon counter (131) and an integrator; and
c) a computer and a processor,
wherein said excitation illumination (132) and said multi-spectral emission detector (134) are disposed on the same side of said cartridge (150); and wherein said optics unit (130) is adapted to receive and detect via a portion of said pathway a plurality of spectrally distinct signals generated by interaction of said radiation and said pre-treated sample in said sample excitation/interaction section (156), wherein said computer is adapted to receive data related to said plurality of spectrally distinct signals and wherein said processor is adapted to process this data, thereby determining said medical condition.

2. A system according to claim 1, wherein said processor is further adapted to output at least one output related to said medical condition.

3. A system according to claim 1, wherein each signal of said plurality of spectrally distinct signals is associated with at least one predetermined biological marker.

4. A system according to claim 1, wherein said pre-analytical sample processing section comprises (154) at least one of the following:
i. an incubator element adapted to incubate said body fluid with at least one antibody;
ii. an incubator element adapted to incubate said body fluid with at least one antigen;
iii. a first mixing element adapted to mix at least one cell type in the body fluid with at least one of a stain and a marker to form at least one of a stained cell type and a marked cell type;
iv. a reacting element adapted to react at least one cell type of said body fluid with said at least one reactant to form said pre-treated sample;
v. a thermal element adapted to heat or cool at least part of said bodily fluid;
vi. a second mixing element adapted to mix at least one cell type in the body fluid with at least one reference material; and
vii. reaction element adapted to form a chemical reaction between said at least one reactant and at least one element of said body fluid.

5. A system according to claim 1, wherein said sample excitation/interaction section (156) comprises a thin metallic lining layer.

6. A system according to claim 1, wherein said least one excitation illumination comprises (132) at least one of a laser, and light emitting diode (LED) and an arc lamp.

7. A system according to claim 1, wherein said pre-treated sample comprises particles adapted to flow in an essentially single-file manner past said radiation.

8. A system according to claim 1, wherein said medical condition is sepsis, and wherein said body fluid comprises white blood cells and said at least one reactant is adapted to detect CD64.

9. A system according to claim 1, wherein said at least one reactant comprises multiple fluorescently-tagged antibodies specific for biological markers.

10. A method for analyzing particles associated with body fluid, the method comprising:
a) providing a multi-sectioned disposable cartridge comprising:
i. a body fluid aspiration section (152) adapted to receive the body fluid directly or indirectly from a patient;
ii. a pre-analytical sample processing section (154) adapted to react at least one reactant with said bodily fluid to form a pre-treated sample;
iii. a sample excitation/interaction section (156);
iv. fluid conveying means for conveying said pre-treated sample from said pre-analytical sample processing section to said sample excitation/interaction section; and
v. a spent sample disposal section (158);
b) reacting at least one reactant with a bodily fluid in the pre-analytical sample processing section (154) in the multi-sectioned disposable cartridge (150) to form a pre-treated sample;
c) conveying said pre-treated sample from the pre-analytical sample processing section (154) to the sample excitation/interaction section (156);
d) impinging radiation, from an optics unit (130) via a pathway, said pre-treated sample disposed in said sample excitation/interaction section (156) in said disposable cartridge (150) thereby generating a plurality of spectrally distinct signals in the direction of at least one multi-spectral emission detector (134) in said optics unit (130); and
e) detecting said plurality of spectrally distinct signals partially via said pathway in said optics unit generated by interaction of said radiation and said pre-treated sample disposed in said sample excitation/interaction section (156).

11. A method according to claim 10, wherein each signal of said plurality of spectrally distinct signals is associated with at least one predetermined biological marker.

12. A method according to claim 11, wherein said reacting step comprises at least one of:
a) receiving the body fluid directly or indirectly from a patient;
b) performing at least one pre-analytical reaction;
c) exciting said pre-treated sample; and
d) disposing said pre-treated sample in a spent sample disposal section.

13. A method according to claim 12, wherein said at least one pre-analytical reaction comprises at least one of the following:
i. incubating said body fluid with at least one antibody;
ii. incubating said body fluid with at least one antigen;
iii. mixing at least one cell type in the body fluid with at least one of a stain and a marker to form at least one of a stained cell type and a marked cell type;
iv. reacting at least one cell type of said body fluid with said at least one reactant to form said pre-treated sample;
v. thermally treating at least part of said bodily fluid;
vi. mixing at least one cell type in the body fluid with at least one reference material; and
vii. forming a chemical reaction between said at least one reactant and at least one element of said body fluid.

14. A method according to claim 10, further comprising performing steps a) to d) on a first sample from a patient.

15. A method according to claim 14, further comprising waiting for a period of time and then repeating steps a) to d) on a further sample.

16. A method according to claim 10, wherein said body fluid comprises less than 100 microliters.

17. A system according to claim 1, further comprising a computer activated by a computer software product.

18. A method according to claim 10, wherein said conveying step comprises conveying particles in said sample to flow in single file through a capillary region past said radiation.

19. A system according to claim 1, wherein said sample excitation/interaction section comprises a capillary section adapted to pass particles in single file through said radiation.

## Patentansprüche

1. Mikro-Durchflusszytometer-System (100, 200) zur Bestimmung eines medizinischen Leidens, wobei das System umfasst:
a) eine in mehrere Abschnitte aufgeteilte wegwerfbare Kassette (150), die daran angepasst ist, dass darin Manipulation und Fluss von Proben stattfinden, wobei die Kassette angepasst ist, ein Volumen einer Körperflüssigkeit aufzunehmen, wobei die Kassette eine Vielzahl von Abschnitten umfasst, umfassend:
i. einen Körperflüssigkeits-Aspirationsabschnitt (152), der angepasst ist, die Körperflüssigkeit direkt oder indirekt von einem Patienten aufzunehmen;
ii. einen voranalytischen-Proben-Verarbeitungsabschnitt (154), der angepasst ist, mindestens einen Reaktanten mit der Körperflüssigkeit reagieren zu lassen, um eine vorbehandelte Probe zu bilden;
iii. einen Proben-Anregungs/Wechselwirkungs-Abschnitt (156);
iv. Flüssigkeitsbeförderungsmittel, zum Befördern der vorbehandelten Probe von dem voranalytischen-Proben-Verarbeitungsabschnitt zu dem Proben-Anregungs/Wechselwirkungs-Abschnitt; und
v. einen Entsorgungsabschnitt (158) für verbrauchte Proben;
b) eine Optikeinheit (130), umfassend:
i. eine Anregungsbeleuchtung (132), die angepasst ist, Strahlung über einen ersten Pfad zu der vorbehandelten Probe über einen Pfad zu befördern;
ii. einen multispektralen Emissionsdetektor (134), der angepasst ist, die Spektralemission in Spektralbändern abzutasten; und
iii. einen Photonenzähler (131) und einen Integrierer; und
c) einen Computer und einen Prozessor,
wobei die Anregungsbeleuchtung (132) und der multispektrale Emissionsdetektor (134) auf der gleichen Seite der Kassette (150) angeordnet sind; und wobei die Optikeinheit (130) angepasst ist, über einen Teilbereich des Pfades eine Vielzahl spektral abgegrenzter Signale aufzunehmen und zu detektieren, die durch Wechselwirkung der Strahlung und der vorbehandelten Probe in dem Proben-Anregungs/Wechselwirkungs-Abschnitt (156) erzeugt wurden, wobei der Computer angepasst ist, Daten aufzunehmen, die sich auf die Vielzahl spektral abgegrenzter Signale beziehen, und wobei der Prozessor angepasst ist, diese Daten zu verarbeiten und damit das medizinische Leiden zu bestimmen.

2. System gemäß Anspruch 1, wobei der Prozessor weiterhin angepasst ist, mindestens eine Ausgabe auszugeben, die sich auf das medizinische Leiden bezieht.

3. System gemäß Anspruch 1, wobei jedes Signal der Vielzahl spektral abgegrenzter Signale mindestens einem vorbestimmten biologischen Marker zugehörig ist.

4. System gemäß Anspruch 1, wobei der voranalytische-Proben-Verarbeitungsabschnitt (154) mindestens eines des Folgenden umfasst:
i. ein Inkubatorelement, das angepasst ist, die Körperflüssigkeit mit mindestens einem Antikörper zu inkubieren;
ii. ein Inkubatorelement, das angepasst ist, die Körperflüssigkeit mit mindestens einem Antigen zu inkubieren;
iii. ein erstes Mischelement, das angepasst ist, mindestens einen Zelltyp in der Körperflüssigkeit mit mindestens einem von einem Farbstoff und einem Marker zu mischen, um mindestens eines von einem gefärbten Zelltyp und einem markierten Zelltyp zu bilden;
iv. ein Reaktionselement, das angepasst ist, mindestens einen Zelltyp der Körperflüssigkeit mit dem mindestens einen Reaktanten reagieren zu lassen, um die vorbehandelte Probe zu bilden;
v. ein thermisches Element, das angepasst ist, mindestens einen Teil der Körperflüssigkeit zu erhitzen oder zu kühlen;
vi. ein zweites Mischelement, das angepasst ist, mindestens einen Zelltyp in der Körperflüssigkeit mit mindestens einem Referenzmaterial zu mischen; und
vii. ein Reaktionselement, das angepasst ist, eine chemische Reaktion zwischen dem mindestens einen Reaktanten und mindestens einem Element der Körperflüssigkeit zu bilden.

5. System gemäß Anspruch 1, wobei der Proben-AnregungslWechselwirkungs-Abschnitt (156) eine dünne metallische Beschichtungslage umfasst.

6. System gemäß Anspruch 1, wobei die mindestens eine Anregungsbeleuchtung (132) mindestens eines von einem Laser, und einer Leuchtdiode (LED) und einer Bogenlampe umfasst.

7. System gemäß Anspruch 1, wobei die vorbehandelte Probe Teilchen umfasst, die angepasst sind, im Wesentlichen eines nach dem anderen an der Strahlung vorüber zu fließen.

8. System gemäß Anspruch 1, wobei es sich bei dem medizinischen Leiden um Sepsis handelt, und wobei die Körperflüssigkeit weiße Blutzellen umfasst und wobei der mindestens eine Reaktant angepasst ist, CD64 zu detektieren.

9. System gemäß Anspruch 1, wobei der mindestens eine Reaktant mehrere fluoreszierend gekennzeichnete Antikörper spezifisch für biologische Marker umfasst.

10. Verfahren zum Analysieren von Teilchen, die einer Körperflüssigkeit zugehörig sind, wobei das Verfahren umfasst:
a) Bereitstellen einer in mehrere Abschnitte aufgeteilten wegwerfbaren Kassette, die umfasst:
i. einen Körperflüssigkeits-Aspirationsabschnitt (152), der angepasst ist, die Körperflüssigkeit direkt oder indirekt von einem Patienten aufzunehmen;
ii. einen voranalytischen-Proben-Verarbeitungsabschnitt (154), der angepasst ist, mindestens einen Reaktanten mit der Körperflüssigkeit reagieren zu lassen, um eine vorbehandelte Probe zu bilden;
iii. einen Proben-Anregungs/Wechselwirkungs-Abschnitt (156);
iv. Flüssigkeitsbeförderungsmittel, zum Befördern der vorbehandelten Probe von dem voranalytischen-Proben-Verarbeitungsabschnitt zu dem Proben-Anregungs/Wechselwirkungs-Abschnitt; und
v. einen Entsorgungsabschnitt (15 8) für verbrauchte Proben;
b) Reagieren mindestens eines Reaktanten mit einer Körperflüssigkeit in dem voranalytischen-Proben-Verarbeitungsabschnitt (154) in der in mehrere Abschnitte aufgeteilten wegwerfbaren Kassette (150), um eine vorbehandelte Probe zu bilden;
c) Befördern der vorbehandelten Probe von dem voranalytischen-Proben-Verarbeitungsabschnitt (154) in den Proben-Anregungs/Wechselwirkungs-Abschnitt (156);
d) Auftreffen lassen von Strahlung, von einer Optikeinheit (130) über einen Pfad, auf die vorbehandelte Probe, die in dem Proben-Anregungs/Wechselwirkungs-Abschnitt (156) in der wegwerfbaren Kassette (150) angeordnet ist, und damit Erzeugen einer Vielzahl spektral abgegrenzter Signale in der Richtung mindestens eines multispektralen Emissionsdetektors (134) in der Optikeinheit (130); und
e) Detektieren, teilweise über den Pfad in der Optikeinheit, der Vielzahl spektral abgegrenzter Signale, die durch Wechselwirkung der Strahlung und der vorbehandelten Probe erzeugt werden, die in dem Proben-Anregungs/Wechselwirkungs-Abschnitt (156) angeordnet ist.

11. Verfahren gemäß Anspruch 10, wobei jedes Signal der Vielzahl spektral abgegrenzter Signale mindestens einem vorbestimmten biologischen Marker zugehörig ist.

12. Verfahren gemäß Anspruch 11, wobei der Reaktionsschritt mindestens eines umfasst von:
a) Aufnehmen der Körperflüssigkeit direkt oder indirekt von einem Patienten;
b) Durchführen mindestens einer voranalytischen Reaktion;
c) Anregen der vorbehandelten Probe; und
d) Anordnen der vorbehandelten Probe in einem Entsorgungsabschnitt für verbrauchte Proben.

13. Verfahren gemäß Anspruch 12, wobei die mindestens eine voranalytische Reaktion mindestens eines des Folgenden umfasst:
i. Inkubieren der Körperflüssigkeit mit mindestens einem Antikörper;
ii. Inkubieren der Körperflüssigkeit mit mindestens einem Antigen;
iii. Mischen mindestens eines Zelltyps in der Körperflüssigkeit mit mindestens einem von einem Farbstoff und einem Marker, um mindestens eines von einem gefärbten Zelltyp und einem markierten Zelltyp zu bilden;
iv. Reagieren mindestens eines Zelltyps der Körperflüssigkeit mit dem mindestens einen Reaktanten, um die vorbehandelte Probe zu bilden;
v. thermisches Behandeln mindestens eines Teils der Körperflüssigkeit;
vi. Mischen mindestens eines Zelltyps in der Körperflüssigkeit mit mindestens einem Referenzmaterial; und
vii. Bilden einer chemischen Reaktion zwischen dem mindestens einen Reaktanten und mindestens einem Element der Körperflüssigkeit.

14. Verfahren gemäß Anspruch 10, weiterhin umfassend Durchführen der Schritte a) bis d) an einer ersten Probe von einem Patienten.

15. Verfahren gemäß Anspruch 14, weiterhin umfassend Warten für eine Zeitspanne und danach Wiederholen der Schritte a) bis d) an einer weiteren Probe.

16. Verfahren gemäß Anspruch 10, wobei die Körperflüssigkeit weniger als 100 Mikroliter umfasst.

17. System gemäß Anspruch 1, weiterhin umfassend einen Computer, der durch ein Computersoftwareprodukt aktiviert wird.

18. Verfahren gemäß Anspruch 10, wobei der Befördungs-Schritt Befördern der Teilchen in der Probe umfasst, um eines nach dem anderen durch einen Kapillarbereich an der Strahlung vorüber fließen zu lassen.

19. System gemäß Anspruch 1, wobei der Proben-Anregungs/Wechselwirkungs-Abschnitt einen Kapillarabschnitt umfasst, der angepasst ist, Teilchen eines nach dem anderen durch die Strahlung zu führen.

## Revendications

1. Système de cytométrie en micro-flux (100, 200) destiné à la détermination d'un état pathologique, le système comprenant :
a) une cartouche jetable à sections multiples (150) adaptée pour avoir une manipulation et un débit d'échantillon qui surviennent dans celle-ci, ladite cartouche étant adaptée pour recevoir un volume d'un fluide corporel, ladite cartouche comprenant une pluralité de sections comprenant :
i. une section d'aspiration de fluide corporel (152) adaptée pour recevoir le fluide corporel directement ou indirectement à partir d'un patient ;
ii. une section de traitement d'échantillon pré-analytique (154) adapté pour faire réagir au moins un réactif avec ledit fluide corporel pour former un échantillon prétraité ;
iii. une section d'excitation/d'interaction d'échantillon (156) ;
iv. un moyen de transport de fluide pour transporter ledit échantillon prétraité à partir de ladite section de traitement d'échantillon pré-analytique à ladite section d'excitation/d'interaction d'échantillon ; et
v. une section d'élimination d'échantillon usé (158) ;
b) une unité optique (130) comprenant :
i. un éclairage d'excitation (132) adapté pour transporter le rayonnement par l'intermédiaire d'une première voie audit échantillon prétraité par l'intermédiaire d'une voie ;
ii. un détecteur d'émission multi-spectral (134) adapté pour échantillonner l'émission spectrale en des bandes spectrales ; et
iii. un d'un compteur de photons (131) et d'un intégrateur ; et
c) un ordinateur et un processeur,
dans lequel ledit éclairage d'excitation (132) et ledit détecteur d'émission multi-spectral (134) sont disposés du même côté de ladite cartouche (150) ; et dans lequel ladite unité optique (130) est adaptée pour recevoir et détecter par l'intermédiaire d'une partie de ladite voie une pluralité de signaux distincts de manière spectrale générée par l'interaction dudit rayonnement et dudit échantillon prétraité dans ladite section d'excitation/d'interaction d'échantillon (156), dans lequel ledit ordinateur est adapté pour recevoir des données relatives à ladite pluralité de signaux distincts de manière spectrale et dans lequel ledit processeur est adapté pour traiter ces données, déterminant ainsi ledit état pathologique.

2. Système selon la revendication 1, dans lequel ledit processeur est en outre adapté pour donner au moins un résultat relatif audit trouble pathologique.

3. Système selon la revendication 1, dans lequel chaque signal de ladite pluralité de signaux distincts de manière spectrale est associé avec au moins un marqueur biologique prédéterminé.

4. Système selon la revendication 1, dans lequel ladite section de traitement d'échantillon pré analytique (154) comprend au moins un des éléments suivants :
i. un élément d'incubateur adapté pour incuber ledit fluide corporel avec au moins un anticorps ;
ii. un élément d'incubateur adapté pour incuber ledit fluide corporel avec au moins un antigène ;
iii. un premier élément de mélange adapté pour mélanger au moins un type cellulaire dans le fluide corporel avec au moins un d'une tâche et d'un marqueur pour former au moins un d'un type cellulaire taché et d'un type cellulaire marqué ;
iv. un élément de réaction adapté pour faire réagir au moins un type cellulaire dudit fluide corporel avec ledit au moins un réactif pour former ledit échantillon prétraité ;
v. un élément thermique adapté pour chauffer ou refroidir au moins une partie dudit fluide corporel ;
vi. un second élément de mélange adapté pour mélanger au moins un type cellulaire dans le fluide corporel avec au moins une matière de référence ; et
vii. un élément de réaction adapté pour former une réaction chimique entre ledit au moins un réactif et au moins un élément dudit fluide corporel.

5. Système selon la revendication 1, dans lequel la section d'excitation/d'interaction d'échantillon (156) comprend une fine couche de doublure métallique.

6. Système selon la revendication 1, dans lequel ledit au moins un éclairage d'excitation (132) comprend au moins un d'un laser, et d'une diode électroluminescente (DEL) et d'une lampe à arc.

7. Système selon la revendication 1, dans lequel ledit échantillon pré traité comprend des particules adaptées pour s'écouler essentiellement en file indienne au-delà dudit rayonnement.

8. Système selon la revendication 1, dans lequel ledit état pathologique est la septicémie, et dans lequel ledit fluide corporel comprend des globules blancs et ledit au moins un réactif est adapté pour détecter CD64.

9. Système selon la revendication 1, dans lequel ledit au moins un réactif comprend de multiples anticorps marqués de manière fluorescente spécifiques à des marqueurs biologiques.

10. Procédé d'analyse de particules associées avec un fluide corporel, le procédé consistant à :
a) fournir une cartouche jetable à sections multiples comprenant :
i. une section d'aspiration de fluide corporel (152) adaptée pour recevoir le fluide corporel directement ou indirectement à partir d'un patient ;
ii. une section de traitement d'échantillon pré-analytique (154) adapté pour faire réagir au moins un réactif avec ledit fluide corporel pour former un échantillon prétraité ;
iii. une section d'excitation/d'interaction d'échantillon (156) ;
iv. un moyen de transport de fluide pour transporter ledit échantillon prétraité à partir de ladite section de traitement d'échantillon pré-analytique vers ladite section d'excitation/d'interaction d'échantillon ; et
v. une section d'élimination d'échantillon usé (158) ;
b) faire réagir au moins réactif avec un fluide corporel dans la section de traitement d'échantillon pré-analytique (154) dans la cartouche jetable à sections multiples (150) pour former un échantillon prétraité ;
c) transporter ledit échantillon prétraité à partir de la section de traitement d'échantillon pré-analytique (154) vers la section d'excitation/d'interaction d'échantillon (156) ;
d) envoyer un rayonnement, à partir d'une unité optique (130) par l'intermédiaire d'une voie, sur ledit échantillon prétraité disposé dans ladite section d'excitation/d'interaction d'échantillon (156) dans ladite cartouche jetable (150) générant ainsi une pluralité de signaux distincts de manière spectrale dans la direction d'au moins un détecteur d'émission multi-spectral (134) dans ladite unité optique (130) ; et
e) détecter ladite pluralité de signaux distincts de manière spectrale partiellement par l'intermédiaire de ladite voie dans ladite unité optique générés par interaction dudit rayonnement et dudit échantillon prétraité disposé dans ladite section d'excitation/d'interaction d'échantillon (156).

11. Procédé selon la revendication 10, dans lequel chaque signal de ladite pluralité de signaux distincts de manière spectrale est associée avec au moins un marqueur biologique prédéterminé.

12. Procédé selon la revendication 11, dans lequel ladite étape de réaction comprend au moins un des points suivants consistant à :
a) recevoir le fluide corporel directement ou indirectement à partir d'un patient ;
b) effectuer au moins une réaction pré-analytique ;
c) exciter ledit échantillon prétraité ; et
d) disposer dudit échantillon prétraité dans une section d'élimination d'échantillon usé.

13. Procédé selon la revendication 12, dans lequel ladite au moins une réaction pré analytique comprend au moins un des points suivants consistant à :
i. incuber ledit fluide corporel avec au moins un anticorps ;
ii. incuber ledit fluide corporel avec au moins un antigène ;
iii. mélanger au moins un type cellulaire dans le fluide corporel avec au moins une d'une tâche et d'un marqueur pour former au moins un d'un type cellulaire taché et d'un type cellulaire marqué ;
iv. faire réagir au moins un type cellulaire dudit fluide corporel avec ledit au moins un réactif pour former ledit échantillon prétraité ;
v. traiter de manière thermique au moins une partie dudit fluide corporel ;
vi. mélanger au moins un type cellulaire dans le fluide corporel avec au moins une matière de référence ; et
vii. former une réaction chimique entre ledit au moins un réactif et au moins un élément dudit fluide corporel.

14. Procédé selon la revendication 10, comprenant en outre l'exécution des étapes a) à d) sur un sur un premier échantillon d'un patient.

15. Procédé selon la revendication 14, comprenant en outre l'attente pendant une période de temps et ensuite la répétition des étapes a) à d) sur un échantillon supplémentaire.

16. Procédé selon la revendication 10, dans lequel ledit fluide corporel comprend moins de 100 microlitres.

17. Système selon la revendication 1, comprenant en outre un ordinateur activé par un produit logiciel informatique.

18. Procédé selon la revendication 10, dans lequel ladite étape de transport comprend des particules de transport dans ledit échantillon pour s'écouler en file indienne à travers une région capillaire au-delà dudit rayonnement.

19. Système selon la revendication 1, dans lequel ladite section d'excitation/d'interaction d'échantillons comprend une section capillaire adaptée pour passer des particules en file indienne à travers ledit rayonnement.
